# EUROPEAN PATENT APPLICATION

(11) **EP 2 363 120 A1**
(43) Date of publication of application: **07.09.2011**
(21) Application number: 11154578.6
(22) Date of filing: 15.02.2011
(51) Int. Cl.: A61K 31/13, A61K 31/437, A61P 25/00, A61P 29/00, A61P 37/08, A61P 3/10

(54) **Combinations of dimebolin and memantine**

(30) Priority: 15.02.2010 TR 201001117
(71) Applicant: Sanovel Ilac Sanayi ve Ticaret A.S., 34398 Istanbul (TR)
(72) Inventor: Toksöz, Ahmet, 34398, Istanbul (TR); Cifter, Ümit, 34398, Istanbul (TR); Türkyilmaz, Ali, 34398, Istanbul (TR)
(74) Representative: Sevinç, Erkan

(57) **Abstract**

A pharmaceutical combination, characterized by comprising dimebolin or a pharmaceutically acceptable salt thereof and memantine or a pharmaceutically acceptable salt thereof.

## Description

### Field of Invention

The present invention relates to combinations comprising dimebolin or a pharmaceutically acceptable salt thereof and memantine or a pharmaceutically acceptable salt thereof.

### Background of Invention

Dimebolin is an antihistaminic agent and a N-methyl d-aspartat (NMDA) receptor agonist. Its chemical designation is 2,3,4,5-tetrahydro-2,8-dimethyl-5-(2-(6-methyl-3-pyridyl)ethyl)-1H-pyrido[4,3-b]indole with the following chemical structure of Formula I.

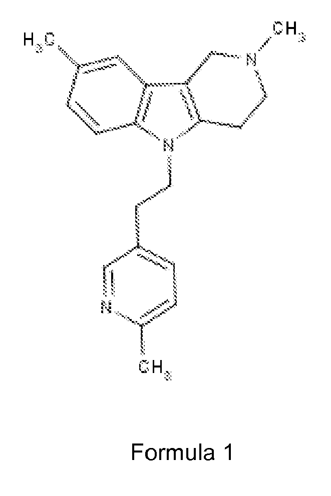

Dimebolin has been in use as an antiallergic agent for more than 20 years. It has been used orally in the form of 2.5 to 20 mg tablets until today. It has been commonly administrated as three tablets per day.

As described in US Patents No 6,187,785 and 7,071,206, hydrogenated pyrido[4,3-b]indole derivatives, such as dimebolin, display NMDA receptor antagonistic features, such features enabling them to be used in treating neurodegenerative diseases, such as the Alzheimer's disease. As specified in WO 2005/055951, hydrogenated pyrido[4,3-b]indole derivatives such as dimebolin has been employed as a geroprotector in humans and pets, or domestic animals. They function by delaying the onset and/or development of age-dependent or age-associated symptoms and/or pathologies or conditions, including disturbance in skin-hair integument, vision disturbance, and weight loss.

U.S. Patent Application No 60/723,403 and U.S. Patent Application No 11/543,529 address hydrogenated pyrido[4,3-b]indole derivatives, such as dimebolin, used as a neuroprotector to treat and/or prevent and/or delay the onset and/or development of Huntington's Disease.

Memantine hydrochloride is a neuroprotective medicament, an N-methyl-D-aspartate (NMDA) receptor antagonist, which is efficacious against dementia. Its chemical designation is 1,3-dimethyl-5-adamantanamine, with the chemical structure illustrated below with Formula 2.

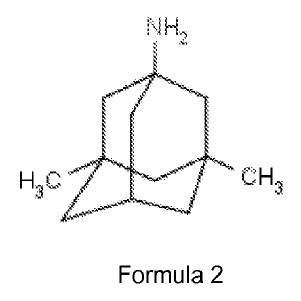

Memantine has already been in used orally, in the form of 5 to 10 mg tablets.

The patent application WO2008005036 discloses an easily-dissolved, granulated memantine formulation.

The patent application WO2007034990 refers to compositions, in which memantine is indicated together with pregabalin or gabapentin.

The patent application US2009124659 refers to a combination of memantine and acetylcholinesterase inhibitors.

There has been no pharmaceutical composition or dosage form produced until today, which contains the combination of dimebolin and memantine. Even if N-methyl d-aspartate (NMDA) receptor agonists and non-competitive N-methyl-D-aspartate (NMDA) receptor antagonists are used together, this fact requires the patients to carry more than one drugs and causes application-related difficulties.

It is known that such drugs used in the same therapeutic field or even for the same symptoms are not brought always together with the expectation of additional therapeutic effects. There are many examples in the past, in which combinations of different classes used to treat the same indications have not yielded efficient or safe dosage forms resulting in different drug combinations. The cause of such unexpected incompatibility is diverse; the typically observed outcomes, however, include increase in side effects of different drug combinations, undesired drug interactions, and formation of new side effects.

Conventional Alzheimer's therapies typically comprise administrating one or more different drug(s) in the form of a pharmaceutical composition. Not all combinations of Alzheimer's drugs, however, are as convenient as they are used individually, with respect to safety and efficiency. In result, a novelty is needed in the art of formulations, which can be used by Alzheimer's patients.

### Object and Brief Description of Invention

The present invention relates to an easily-administrable combination of dimebolin and memantine, eliminating all aforesaid problems and brining additional advantages to the relevant prior art.

Accordingly, the main object of the present invention is to obtain a stable combination formulation with synergistic effect for use in the treatment of Alzheimer's disease.

Another object of the present invention is to obtain a combination of dimebolin and memantine having desired levels of compatibility.

A further object of the present invention is to obtain a combination of dimebolin and memantine having desired levels of bioavailability.

Another object of the present invention is to obtain a combination of dimebolin and memantine having desired levels of dissolution rate and solubility.

In order to achieve all objects, referred to hereinabove and to emerge in the following detailed description, the subject novelty is realized with a combination of dimebolin, or a pharmaceutically acceptable salt thereof, and memantine, or a pharmaceutically acceptable salt thereof.

In a preferred embodiment according to the present invention, said formulation is in the form of a capsule, tablet, powder, granule or sachet.

In a preferred embodiment according to the present invention, said formulation is in the form of a film-coated tablet.

In a preferred embodiment according to the present invention, said combination further comprises an excipient.

In another preferred embodiment according to the present invention, said excipient comprises at least one or a mixture of diluting agents, binders, disintegrants, surface-active agents, glidants, and lubricants.

In another preferred embodiment according to the present invention, said diluents include at least one or a mixture of lactose, microcrystalline cellulose, starch, mannitol, calcium phosphate anhydrate, calcium phosphate dihydrate, calcium phosphate trihydrate, dibasic calcium phosphate, silicium dioxide, glucose.

In another preferred embodiment according to the present invention, said binders include at least one or a mixture of polymethacrylate, glyceryl behenate, polyvinylpyrrolidone (povidone), cross-linked polyvinylpyrrolidone, cellulose derivatives such as hydroxypropyl methylcellulose (HPMC), hydroxypropyl cellulose (HPC), carboxymethyl cellulose (CMC), methyl cellulose (MC), ethyl cellulose, polyethylene oxide, gelatin.

In another preferred embodiment according to the present invention, said disintegrants comprise at least one or a mixture of croscarmellose sodium, sodium starch glycolate, crospovidone, starch (e.g. corn, potato), alginic acid.

In another preferred embodiment according to the present invention, said surface-active agents comprise at least one or a mixture of sodium lauryl sulfate, polyoxyethylene glycol esters, sulfates containing surface-active agents.

In another preferred embodiment according to the present invention, said glidants comprise at least one or a mixture of colloidal silicone dioxide, talc, aluminum silicate, magnesium silicate.

In another preferred embodiment according to the present invention, said lubricants comprise at least one or a mixture of calcium stearate, magnesium stearate, mineral oil, sodium stearyl fumarate, talc, polyethylene glycol, stearic acid.

In another preferred embodiment according to the present invention, said coating agent comprises hydroxypropyl methylcellulose, polyethylene glycol, talc, and titanium dioxide.

In another preferred embodiment according to the present invention, the subject formulation's stability and compatibility are ensured with cellulosic polymer and titanium dioxide which is included in the coating layer.

In another preferred embodiment according to the present invention, said cellulosic polymer is preferably hydroxypropyl methylcellulose.

Another aspect of the present invention provides a method for preparing the subject pharmaceutical composition, this method comprising the steps of
a. adding starch to dimebolin and memantine, mixing the resulting mixture,
b. adding polyvinylpyrrolidone and cross-linked polyvinylpyrrolidone to the resulting mixture, mixing the latter,
c. producing a wet granulation,
d. drying the granules,
e. sieving dried granules,
f. mixing the granules with magnesium stearate and silicium dioxide,
g. compressing the granules into tablets, and
h. film-coating the tablets with a water-soluble agent.

A further aspect of the present invention provides another method for preparing said pharmaceutical combination, this method comprising the steps of
a. adding lactose, polyvinylpyrrolidone, silicium dioxide, and magnesium stearate to dimebolin and memantine, mixing the resulting mixture,
b. compressing the granules into tablets, and
c. film-coating the tablets with a water-soluble agent.

Another aspect of the present invention provides a further method for preparing said pharmaceutical combination, this method comprising the steps of
a. adding lactose monohydrate, polymethacrylate, glyceryl behenate, and hydroxypropyl methylcellulose to dimebolin and memantine, mixing the resulting mixture,
b. forming a wet granulation with a solvent,
c. drying the granules,
d. sieving dried granules,
e. mixing magnesium stearate together with granules,
f. compressing the granules into tablets, and
g. film-coating the tablets with a water-soluble agent.

### Detailed Description of Invention

### Example 1: Film-coated tablet

### Wet Granulation

| **Content** | **weight % (w/w)** |
|---|---|
| **Core tablet** | |
| Dimebolin | 1 - 25 |
| Memantine | 1 - 15 |
| Polyvinylpyrrolidone | 0,5 - 10 |
| Starch | 40 - 85 |
| Cross-linked polyvinylpyrrolidone | 2 - 10 |
| Silicium dioxide | 0,1 - 2 |
| Magnesium stearate | 0,1 - 1,5 |

| **Film Tablet** | |
|---|---|
| Opadry | 2 - 10 |

In the tablet form according to the present invention, starch is added to dimebolin and memantine, and the resulting mixture is mixed. Polyvinylpyrrolidone and cross-linked polyvinylpyrrolidone are added to and mixed together with the resulting mixture, thereby a wet granulation is formed. The formed granules are dried, sieved; thereafter silicium dioxide and magnesium stearate are added therein and mixed together. The formed granules are compressed into tablets. At the final step, tablets are subjected to film-coating.

### Direct compression

| **Content** | **weight % (w/w)** |
|---|---|
| **Core tablet** | |
| Dimebolin | 1 - 25 |
| Memantine | 1 - 15 |
| Lactose (spray-dried) | 40 - 85 |
| Cross-linked polyvinylpyrrolidone | 2 - 10 |
| Silicium dioxide | 0,1 - 2 |
| Magnesium stearate | 0,1 - 1,5 |

| **Film Tablet** | |
|---|---|
| Opadry | 2 - 10 |

In the tablet form according to the present invention, which is made by means of direct-compression, lactose, polyvinylpyrrolidone, silicium dioxide, and magnesium stearate are added to dimebolin and memantine, and the resulting mixture is mixed. At the final step, tablets are subjected to film-coating.

### Example 2: Film-coated controlled-release tablet

| **Content** | **weight % (w/w)** |
|---|---|
| **Core tablet** | |
| Dimebolin | 1 - 25 |
| Memantine | 1 - 15 |
| Lactose monohydrate | 40 - 85 |
| Polymethacrylate | 3 - 25 |
| Hydroxypropyl methylcellulose K100-MCR | 3 - 25 |
| Glyceryl behenate | 5 - 25 |
| Magnesium stearate | 0,3 - 2 |

| **Film Tablet** | |
|---|---|
| Opadry | 2 - 10 |

In the controlled-release tablet form according to the present invention, lactose monohydrate, polymethacrylate, hydroxypropyl methylcellulose, glyceryl behenate are added to dimebolin and memantine, and the resulting mixture is mixed. Then a proper solvent is added so that a wet granulation is obtained from the mixture. The formed granules are dried, sieved; thereafter magnesium stearate is added therein and mixed together. The formed granules are compressed into tablets. At the final step, tablets are subjected to film-coating.

Dimebolin and memantine are used in amounts of 2,5 to 20 mg and 5 to 10 mg, respectively, in various combination formulations according to the present invention, which must be deemed illustrative, but not limiting.

Memantine and dimebolin combination formulations which have surprisingly good bioavailability synergistic effect and good stability are obtained by our invention. Memantine hydrochloride is preferably used in the formulation.

The combinations made according to the present invention can be used to treat Alzheimer's disease, dementia, neuropathic pain, diabetic neuropathy, chronic visceral pain, chronic inflammatory pain, headache and antihistaminic disease.

In Alzheimer's disease, the probability that a patient forgets a second drug is higher than he/she forgets a single drug. Therefore, the use of a single dose with two active ingredients provides convenience with respect to the patient.

Coated oral solid pharmaceutical combination may include coated tablet, coated powder, coated granule.

Coating layer may be functional or non-functional.

Suitable coating agents include, but are not restricted to polyvinylpyrrolidone (PVP), hydroxypropyl cellulose, lowsubstituted hydroxypropyl cellulose, hydroxypropylmethyl cellulose, sodium carboxymethyl cellulose, methyl and ethyl cellulose, hydroxyethyl methylcellulose, polyethylene glycol (PEG), PVP/vinyl acetate copolymer, PVA/PEG copolymer, alginates, sugar, starch, sugar alcohols, starch or mixtures thereof, polyvinyl alcohol like polymers, and all kinds of OpadryTM, and all kinds of EUDRAGIT, as well as pigments, dyes, titanium dioxide and iron oxide, talk, hypromellose, methacrylic acid, methacrylic acid copolymer, cellulose acetate phthalate, cellulose acetate succinate, hydroxpropyl cellulose phthalate, hydroxpropyl ethylcellulose phthalate, hydroxyl propyl methyl cellulose phthalate, hydroxyl propyl methyl cellulose acetate succinate, hydroxyethyl cellulose phthalate, methylcellulose phthalate, polyvinyl acetate phthalate, polyvinylacetate hydrogen phthalate, amylase acetate phthalate, cellulose ester phthalates, cellulose ether phthalates, sodium cellulose acetate phthalate, starch acid phthalate, cellulose acetate butyrate, cellulose acetate maleate, cellulose acetate trimellitate, cellulose acetate propionate, styrene maleic acid dibutyl phthalate copolymer, styrene maleic acid polyvinyl acetate phthalate copolymer propionate, shellac or mixtures thereof, carnauba wax, cellulose acetate, ceresin, cetyl alcohol, chitosan, gelatin, glycerin, glyceryl behenate, glyceryl palmitostearate, hypromellose phthalate, isomalt, latex particles, liquid glucose, maltitol, maltodextrin, microcrystalline wax, paraffin, poloxamer, polydextrose, polyethylene oxide, poly-DL-(lactic acid), shellac, shellac with stearic acid, sucrose, surface color agents, white wax, xylitol, yellow wax, zein.

The protection scope of the present invention is set forth in the annexed Claims and cannot be restricted to the illustrative disclosures given above, under the detailed description. Any alternative embodiments to be produced by persons skilled in the art according to the basic principles, which are under the protection scope as set forth in the Claims, shall be an infringement of the present invention.

## Claims

1. A coated oral solid pharmaceutical combination, **characterized by** comprising dimebolin or a pharmaceutically acceptable salt thereof and memantine or a pharmaceutically acceptable salt thereof.

2. The coated oral solid combination according to Claim 1, this combination being in the form of capsule, tablet, powder, granule, or sachet.

3. The coated oral solid pharmaceutical combination according to claims 1 and 2, this combination being preferably in the form of a film-coated tablet.

4. The coated oral solid pharmaceutical combination according to any of the preceding claims, further comprising at least one excipient.

5. The coated oral solid pharmaceutical combination according to any of the preceding claims, wherein said excipient is at least one or a mixture of diluting agents, binders, disintegrants, surface-active agents, glidants, and lubricants.

6. The coated oral solid pharmaceutical combination according to any of the preceding claims, wherein said diluting agents comprise at least one or a mixture of lactose, microcrystalline cellulose, starch, mannitol, calcium phosphate anhydrate, calcium phosphate dihydrate, calcium phosphate trihydrate, silicium dioxide, and glucose.

7. The coated oral solid pharmaceutical combination according to any of the preceding claims, wherein said binders comprise at least one or a mixture of polymethacrylate, glyceryl behenate, polyvinylpyrrolidone (povidone), cross-linked polyvinylpyrrolidone, cellulose derivatives such as hydroxypropyl methylcellulose (HPMC), hydroxypropyl cellulose (HPC), carboxymethyl cellulose (CMC), methyl cellulose (MC), ethyl cellulose, polyethylene oxide, and gelatin.

8. The coated oral solid pharmaceutical combination according to any of the preceding claims, wherein said disintegrants comprise at least one or a mixture of croscarmellose sodium, sodium starch glycolate, crospovidone, starch, and alginic acid.

9. The coated oral solid pharmaceutical combination according to any of the preceding claims, wherein said surface-active agents comprise at least one or a mixture of sodium lauryl sulfate, polyoxyethylene glycol esters, sulfates containing surface-active agents.

10. The coated oral solid pharmaceutical combination according to any of the preceding claims, wherein said glidants comprise at least one or a mixture of colloidal silicone dioxide, talc, aluminum silicate, and magnesium silicate.

11. The coated oral solid pharmaceutical combination according to any of the preceding claims, wherein said lubricants comprise at least one or a mixture of calcium stearate, magnesium stearate, mineral oil, sodium stearyl fumarate, talc, polyethylene glycol, and stearic acid.

12. The coated oral solid pharmaceutical combination according to any of the preceding claims, wherein said coating agent comprises hydroxypropyl methylcellulose, polyethylene glycol, and titanium dioxide.

13. The coated oral solid pharmaceutical combination according to any of the preceding claims, comprising cellulosic polymer in the core and titanium dioxide at the coating layer.

14. The coated oral solid pharmaceutical combination according to any of the preceding claims, wherein said cellulosic polymer is preferably hydroxypropyl methylcellulose.

15. A method for preparing a coated oral solid pharmaceutical combination according to any of the preceding claims, comprising the steps of
a. adding starch to dimebolin and memantine, mixing the resulting mixture,
b. adding polyvinylpyrrolidone and cross-linked polyvinylpyrrolidone to the resulting mixture, mixing the latter,
c. producing a wet granulation,
d. drying the granules,
e. sieving dried granules,
f. mixing the granules with magnesium stearate and silicium dioxide,
g. compressing the granules into tablets, and
h. film-coating the tablets with a water-soluble agent.

16. A method for preparing a coated oral solid pharmaceutical combination according to any of the preceding claims, comprising the steps of
a. adding lactose, polyvinylpyrrolidone, silicium dioxide, and magnesium stearate to dimebolin and memantine, mixing the resulting mixture,
b. compressing the granules into tablets, and
c. film-coating the tablets with a water-soluble agent.

17. A method for preparing a coated oral solid pharmaceutical combination according to any of the preceding claims, comprising the steps of
a. adding lactose monohydrate, polymethacrylate, glyceryl behenate, and hydroxypropyl methylcellulose to dimebolin and memantine, mixing the resulting mixture,
b. forming a wet granulation with a solvent,
c. drying the granules,
d. sieving dried granules,
e. mixing magnesium stearate together with granules,
f. compressing the granules into tablets, and
g. film-coating the tablets with a water-soluble agent.

18. Use of a coated oral solid pharmaceutical combination according to any of the preceding claims in the production of a drug for treating Alzheimer's disease, dementia, neuropathic pain, diabetic neuropathy, chronic visceral pain, chronic inflammatory pain, headache, and antihistaminic diseases.

19. The oral coated solid pharmaceutical combination according to any of the preceding claims for preventing or treating Alzheimer's disease, dementia, neuropathic pain, diabetic neuropathy, chronic visceral pain, chronic inflammatory pain, headache, and antihistaminic diseases in mammalians and particularly in humans.
